(19)

Europäisches Patentamt

European Patent Office

Office européen des brevets

(11) **EP 3 790 017 A1**

(12) **EUROPEAN PATENT APPLICATION**

(43) Date of publication:
**10.03.2021 Bulletin 2021/10**

(51) Int Cl.:
***G16H 20/10*** *(2018.01)*

(21) Application number: **19196291.9**

(22) Date of filing: **09.09.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bencard Allergie GmbH
80804 München (DE)**

(72) Inventors:
- **Wolfrath, Iris
  80804 München (DE)**
- **Kreis, René
  80804 München (DE)**

(74) Representative: **Nordmeyer, Philipp Werner
df-mp Dörries Frank-Molnia & Pohlman
Patentanwälte Rechtsanwälte PartG mbB
Theatinerstraße 16
80333 München (DE)**

(54) **METHOD AND DEVICE FOR ADHERENCE MONITORING**

(57) The present invention pertains to a method and device for monitoring an adherence of a patient, e.g. for patients requiring and/or undergoing an immunotherapy. Accordingly, a method for monitoring an adherence of a patient is disclosed, comprising the steps of: inputting at least one patient parameter into an evaluation unit, said parameter comprising an indication of a performed treatment (10); determining adherence compliance course (A) by evaluating, in the evaluation unit (20), the inputted parameter over a predefined period of time and comparing the determined compliance course (A) with a predefined compliance course (C); obtaining, by the evaluation unit (10), at least one measurement indicative of a disease status of the patient (14); and outputting a signal (16), if a deviation of the determined compliance course (A) from the predefined compliance course (C) exceeds a threshold (D) and the measurement indicative of a disease status of the patient (14) exceeds a predefined tolerance limit (E).

Fig. 1

EP 3 790 017 A1

**Description**

Technical Field

The invention relates to a method and device for adherence monitoring of a patient, in particular for patients requiring and/or undergoing a long-term therapy, such as e.g. an immunotherapy for allergy patients.

Technological Background

Patients receiving a therapy for the treatment of a particular disease may require a regular administration of e.g. a drug, for example, on a day-to-day basis or periodically between predefined time points. In order to ensure that a therapeutic effect is provided, cooperation of the patient with the prescribed therapy by performing the required therapeutic step is essential in order to maintain or induce a physiological state of the patient and to maximize the efficacy of the treatment. Such cooperation of the patient has previously been assessed by means of a compliance of the patient and is more recently determined by means of an adherence, which also includes the responsibility of the physician in ensuring that a required therapy is performed.

Generally, the level of therapeutic adherence reduces over time, in particular in the case of chronic diseases, such that according to the World Health Organization (Eduardo Sabate: Adherence to long-term therapies: evidence for action, World Health Organization 2003) on average only fifty percent of the patients are treated with the prescribed therapy after about one year. A main factor influencing the level of adherence lies in a lack of sufficient communication of the relevant information to the patient, such that the probability of a reduced compliance of a patient may increase, when a patient is not sufficiently informed about its current health status and the benefits or advantages of adherence to the prescribed therapy.

The adherence or compliance rate may furthermore decline when therapies are prescribed that are to be performed over a plurality of years and wherein a therapeutic effect may not be instantaneously achieved upon each of a series of respective treatments, e.g. in the case of cardiovascular diseases, respiratory diseases, diabetes management, or allergies, wherein the physiological state of the patient generally requires a certain amount of time to recover and/or adapt to the prescribed therapy. For example, diabetes patients suffering from diabetes mellitus type 1 generally require a life-long substitution therapy with insulin. In such cases, a high level of compliance may be essential so as to avoid or delay permanent organ damage. Also, patients suffering from allergies specific for one or more inhalative allergens such as pollen may require a specific immune therapy, which may last up to three to five years, wherein the desired therapeutic effect may not be achieved in the first few months or even years of therapy, as such therapy is generally based on hyposensibilization. In addition, although if an obvious effect is seen in the first few months, a continuation of immunotherapy is needed for a long-lasting effect.

Furthermore, the compliance rate may not only be dependent on a duration of the therapy, but also on the type of therapy. For example, hyposensibilization may be provided in the form of a subcutaneous immune therapy (SCIT) or as a sublingual immune therapy (SLIT), e.g. by means of minimally invasive injections or by the administration of a medicament in the form of droplets, wherein the therapy intervals may vary between said types. The intervals may be subject to patient-specific factors, such as obliviousness of the patient.

In addition, the immune therapy may trigger various physiological reactions, which may be experienced by a patient as undesirable side effects. By the same token, atmospheric increases of allergen or pollen concentrations may induce symptoms of allergic reactions while the hyposensibilization of the patient is ongoing. Accordingly, the patient's therapeutic acceptance may be reduced, leading to a further lowering of the compliance, thereby also further reducing the efficacy of the immune therapy.

Accordingly, there is a need to improve the adherence of the patient to a particular therapy and to assess a patient's health risk due to a reduced adherence.

Summary of the invention

It is an object of the present invention to provide for an adherence monitoring of a patient, in particular for patients requiring and/or undergoing a long-term therapy, such as e.g. an immunotherapy.

In a first aspect, a method for monitoring an adherence of a patient is suggested, which comprises the steps of

- inputting at least one patient parameter into an evaluation unit, said parameter comprising an indication of a performed treatment;

- determining a compliance course by evaluating, in the evaluation unit, the inputted parameter over a predefined period of time and comparing the determined compliance course with a predefined compliance course;

- obtaining, by the evaluation unit, at least one measurement indicative of a disease status of the patient; and

- outputting a signal, if a deviation of the determined compliance course from the predefined compliance course exceeds a threshold and/or the measurement indicative of a disease status of the patient exceeds a predefined tolerance limit.

The treatment may be an immunization treatment, e.g. to provide a therapy against one or more allergies of the patient, wherein the measurement indicative of a disease status of the patient may be e.g. a physiological parameter of the patient and/or an atmospheric pollen concentration.

In the case of such allergy therapy, the performed immunization treatment may comprise a subcutaneous immune therapy, wherein a dosage of one or more allergens specific for one or more types of pollen are administered to the patient via the skin by means of a minimally invasive injection. Administration of the medication may be performed by a physician, e.g. at a medical facility or institution, but also may be provided in the form of self-medication, e.g., in the form of tablets or drops when providing an oral or sublingual immune therapy. Alternatively, the immunization treatment may be performed epicutaneously. Accordingly, a patient itself may take in the required immunization treatment, independent of its current location and independent of the presence of medical assistance. Such therapy may span over prolonged periods of times, e.g. between three to five years, such that the number of treatments as well as the intervals may vary.

The method for monitoring an adherence of a patient is not limited to a particular treatment or therapy such as an immunization treatment and may hence instead be applied to any medical treatment or therapy in which a patient is required to perform a medical treatment or take in a dosage of a medicament on a regular or semi-periodical basis.

Accordingly, the inputted information that has to include at least one patient parameter may comprise an indication of a performed medical treatment or intake of a medicament, drug, or medical product and the measurement indicative of a disease status of the patient may comprise at least one measurement of a physiological parameter of the patient.

The inputted patient parameter may e.g. indicate that a medicament has been taken and may furthermore comprise a type of medicament, a dosage, and a timestamp indicating the time point the medicament has been taken. The medicament and dosage may correspond to a patient's prescription and/or may vary over time, e.g. may need to be adapted over time by a physician, depending on the patient's symptoms.

For example, a patient may chronically suffer from hypertension or high blood pressure and may require an intake of e.g. diuretics, beta-blockers, and/or ACE-inhibitors, yet may not require all medicaments at the same time or for the same symptoms, since e.g. diuretics are mainly intended to reduce the right ventricular load and remove excess fluid being retained in the patient's body. In contrast, beta-blockers directly affect the triggering and frequency of the heartbeat and may generally reduce a vascular tonus, similar to ACE-inhibitors.

By inputting whether the intake of a particular medicament has been performed a compliance course may be determined by evaluating this information over a predefined period of time and by comparing this information with a predefined compliance course. For example, the information may indicate that a particular treatment has been delayed or has been performed with a deviating medicament dosage compared with a patient prescription. The predefined compliance course, however, is not required to fully correspond with the patient prescription, but may also factor in a temporal delay or dosage tolerance, such that the predefined compliance course is not required to reflect an ideal situation.

Furthermore, at least one measurement of a physiological parameter of the patient is obtained, which may be received from a patient database or a peripheral device, or may be actively measured by the patient, e.g., in the form of a cuff to measure a blood pressure of the patient. In such case, a signal may be outputted, if a deviation of the determined compliance course from the predefined compliance course exceeds a threshold and/or the blood pressure measurement exceeds a predefined tolerance limit. For example, if a patient repeatedly forgets to take in the appropriate medication to control the blood pressure and/or takes in the wrong medicament dosage while at the same time the blood pressure of the patient exceeds a predefined value, e.g. 180 mmHg, a signal may be outputted to indicate the necessity of a medical treatment and/or to avoid any medical complications that may provide a health risk or put a burden on the patient.

By the same token, the method for monitoring an adherence of a patient may be applied to a patient suffering from a cardiovascular disease, wherein the medicament may e.g. be a beta-blocker. Furthermore, the measurement of a physiological parameter of the patient may comprise e.g. a pulse or heartbeat frequency, which may be detected by a wearable device such as e.g. a smart watch, or fitness tracker. The measurements may also comprise other signals derived from an obtained ECG measurement, e.g., P wave, PR interval, QRS complex, J-point, ST segment and T wave, such that, in addition to a heart frequency, also further information may be derived. Such measurements may be provided e.g. by a wearable ECG device or may alternatively be received from a coupled external heart monitor. In addition, the measurements may also be obtained during activity of the patient, e.g. in the form of an exercise ECG. Accordingly, a reduced compliance may result in cardiac irregularities, such that a signal may be outputted, when a deviation of the determined compliance course from the predefined compliance course exceeds a threshold and/or e.g. characteristics of the ECG signal exceed a predefined tolerance limit.

In a further embodiment, the method for monitoring an adherence of a patient may be applied to a patient suffering from diabetes, wherein the medical treatment comprises the injection of insulin. Depending on the type of insulin, the insulin may either be slowly released over a prolonged period of time or may have an immediate effect on the blood sugar level of the patient, e.g., in the case of a hyperglycemic condition. When the long-term insulin is not administered at predefined time points, this may reduce the efficacy of the treatment, as doses may overlap or may be insufficient, such that the patient is required to either increase the sugar consumption, e.g., in a hypoglycemic situation, or increase the dosage of short-term insulin, e.g., in a hyperglycemic situation.

Accordingly, although the patient's condition may be alleviated or remedied, a repeated occurrence of such delay may cause vascular diseases, which may have further consequences for end organs, such as the heart and kidneys of the patient. Therefore, the monitoring of the adherence or compliance together with a regular or periodic blood sugar measurement, which may be performed by a portable blood sugar meter and a corresponding lancet, may result in an outputted signal indicative of a necessary medical treatment and the importance of complying with the prescribed administration of the medicament, e.g. by means of a corresponding signal.

In still a further embodiment, the method for monitoring an adherence of a patient may be applied to a patient suffering from respiratory diseases, wherein the medicament may be an inhalable medicament to e.g. dilate the bronchi and bronchioles and/or reduce inflammation and edema. Furthermore, medicaments for counteracting an allergic reaction, such as an antihistamine may be required in certain situations, with no adequate alternative treatment options. Accordingly, the inputting of the patient parameter may include the corresponding information of the medicament that has been administered and may comprise both a type and dosage of the medicament.

In this case, the obtained measurement of a physiologic parameter may be a breathing frequency and/or breathing volume, which may be measured by e.g. a wearable inhaler. Accordingly, when a low compliance is determined and/or an increased breathing frequency with a low breathing volume is determined, a signal may be output indicating that a physical condition of the patient is declining and the health risk is further increased, if a medical treatment is not continued. Furthermore, further measurements relating to e.g. an ambient air temperature, humidity, and/or purity may be obtained, which may be factored into the outputting of the signal.

In addition to the obtained measurement of a physiologic parameter, the adherence monitoring may furthermore be provided with patient parameters relating to a physical activity of the patient, such as a fitness score, a (recreational) sport record, and/or a movement of the patient, e.g. via a health monitoring implementation on a personal device, such as a smart watch or smart phone. Such data may be factored in, when determining if a deviation of the determined compliance course from the predefined compliance course exceeds a threshold and/or the measurement of physiological parameter exceeds a predefined tolerance limit.

Hence, an adherence monitoring is provided that is compatible with a variety of disorders and which may be implemented in a corresponding device.

The inputting of a performed treatment may be provided at a central or remote system, e.g. at a medical facility or practice, yet is advantageously provided on a portable device of the patient, e.g., a mobile phone, PDA, tablet, or notebook via an implemented module. Accordingly, such device may comprise an inputting interface, such as a touch-screen or keypad. The inputting may furthermore be performed between the respective treatments, such that an interval between said treatments may be determined.

The inputted treatments and/or intervals may then be used by the evaluation unit to determine a compliance course in view of a prescribed therapy, wherein a number of treatments are prescribed at particular time intervals or time points. For example, an adherence may be determined based on a time difference between a respective inputted treatment and a prescribed treatment associated with the respective inputted treatment. A patient, may e.g. perform a treatment at the prescribed time point, e.g., in a particular time interval, e.g. a week or couple of days, such that a time difference equals zero, thereby providing a compliance level of 100 percent and being adherent. By the same token, a premature, delayed, or discontinued treatment may result in a lower compliance level and may furthermore affect a compliance course, e.g., in the case of a regular mismatch and/or extended deviation.

By the same token, a predefined compliance course may be provided based on the prescribed therapy, wherein said predefined compliance course may comprise a desirable compliance level, e.g. for a plurality of time points, wherein a tolerance level may be factored into the predefined course, such that the predefined compliance course does not correspond to an optimal compliance, but to a compliance within the range of between e.g. 50 percent to 95 percent, preferably between 50 percent and 70 percent. Such predefined compliance course may furthermore be based on the complexity of the therapy, e.g. whether a sublingual immunotherapy or a subcutaneous immunotherapy is performed, a length of therapy, and/or a frequency of the treatments, e.g. whether said treatments are required perennial, pre-seasonal, or only for a short period of the treatment year. Furthermore, the predefined compliance may be based on patient-specific parameters, such as, e.g., side effects, symptoms, or trust in treatment of the patient.

The evaluation unit furthermore obtains at least one measurement indicative of a disease status of the patient, which in the case of an immunization treatment or therapy may be a measurement of a pollen concentration, so as to provide a measurement of a factor, e.g. an external environmental factor, that is related to and affects the physiological status of a patient, thereby forming a potential indication of a disease status. Such measurement may e.g. be provided by e.g. a weather station, for example, via a central server, or may be provided by means of one or more evaluated actual measurements of pollen concentration received from a central service, such as a pollen warning service, e.g. in Austria, or a pollen information service, e.g. in Germany. Such measurements may be performed by a plurality of pollen traps and may comprise time-dependent and/or region dependent information, wherein the evaluation unit may assess the relevance for a patient based on e.g. the type of pollen, a measurement trend, and/or a residential address of the patient. Thereby, the evaluation unit may assess whether the measurement may pose a health risk, e.g. a risk of increasing symptoms, for a respective patient.

Based on the determined deviation between the determined compliance course and the predefined compliance course, the evaluation unit may output a signal, which may include a prompting to increase the inputting frequency of the patient parameter, e.g. by providing a visual indication on a display and/or an outputted audio signal on a portable and/or mobile device of the patient. Furthermore, a warning signal may be transmitted to a physician to be accordingly output, e.g. by providing a visual indication on a display, an outputted audio signal, e.g. provided by a buzzer, and/or an electromechanical warning signal in the form of a vibration on a stationary or portable and/or mobile monitoring device of the physician.

For example, the determined deviation may indicate that the determined compliance is below an absolute compliance level and/or may indicate that the determined deviation exceeds an absolute threshold. Based on said comparison of the deviation with a threshold and/or by assessing whether the measurement indicative of a disease status of the patient exceeds a predefined tolerance limit, a health risk of the patient may be determined, e.g. a risk of a loss of adherence or of increasing symptoms of an allergic reaction e.g. during a corresponding allergy season. However, the signal or warning signal may also be outputted, when a low health risk is determined, e.g. by a corresponding threshold value. In either case, a physician may be provided with information related to the actual compliance course and/or a comparison to the predefined compliance course, e.g. by providing a prediction of a patient's adherence and/or one or more factors influencing said adherence.

For example, a low health risk may be determined, when the patient has been treated according to a prescribed immunization schedule or therapy for a predefined period of time, e.g. the first months of treatment, even if a measurement of the pollen concentration indicates a moderate risk, since the efficacy of the immunization may provide sufficient protection against the indicated pollen concentration. Accordingly, in such case, a portable device of the patient may indicate that the treatment is up-to-date or that a pollen concentration may increase, yet no warning signal may be outputted to the patient. Instead, a physician may be notified of the increasing status of the pollen concentration and the potential of a patient developing symptoms, such that a reassurance of the efficacy of the performed therapy may be required.. Thereby, a patient may be motivated to maintain the therapy, such that not only the success of the therapy may be tracked, but a reduced compliance may also be avoided.

By the same token, a lower threshold may be provided for an initial phase of the therapy, wherein an exceeding of said threshold may also result in outputting of a signal, even if the measurement indicative of a disease status of the patient poses a low risk of development of symptoms. This is because the therapy, e.g. the immunization, may be insufficient to protect the patient from developing symptoms, e.g. an allergic reaction, such that also low levels of pollen concentrations may affect the patient's health or physical state.

Furthermore, a declining trend of the compliance course and an inclining trend of the measurement indicative of a disease status of the patient, e.g. a pollen concentration, may trigger the outputting of a signal so as to provide a physician with the information regarding an increased health risk of the patient and which may provide references to guidelines or best practice, e.g. to remind the patient of the necessity of the treatment and/or to perform appropriate countermeasures, e.g., in the form of appropriate medicaments and/or by performing a required treatment. Such triggering may e.g. be provided by an absolute change in the respective trend or may be provided by accordingly adjusting the threshold and/or tolerance limit.

In addition, when the patient is also inputting symptoms being experienced, a physician may also accordingly provide the patient with information about which drugs, medicaments, or medical products may be used according to corresponding guidelines so as to remedy or provide a therapy for the experienced symptoms. For example, the patient may be pointed to a use of antihistamines for treating symptoms occurring in the nose and/or eyes of the patient in case of e.g. rhinitis or conjunctivitis or may be pointed to the use of cortisol-based nasal sprays or asthma medication when experiencing symptoms in the lungs, for example.

By determining the compliance course and factoring in the measurement indicative of a disease status of a patient, e.g. a measurement of a pollen concentration, an assessment of a physiological status of the patient is provided and may be continuously updated, so as to provide a monitoring of the patient. Thereby, a current compliance status and compliance course may be determined while at the same time providing a warning system for a physician, which correlates with the current adherence status, i.e. whether a patient is at risk of losing adherence. This not only provides the possibility of an intervention, but also may remind the patient of the importance of its ongoing immunotherapy, so as to improve the acceptance and to increase the compliance or maintain the compliance at the predefined level.

An early recognition of a reduced compliance may be essential to avoid any undesired symptoms and may be remedied by communicating additional information to the patient so as to re-establish the required compliance that is necessary to ensure that the desired therapeutic effect is achieved. Accordingly, the outputted signal may comprise additional patient and therapy information and may concomitantly alert medical personnel of a potential health risk of the patient. Thereby, a loss of adherence may be effectively avoided, even in the absence of an appointment with a medical professional. By the same token, the signal may provide an indication to seek medical assistance or to contact a medical professional, so as to further increase the adherence, if required.

The increase or maintenance of the compliance or adherence may not only improve the patient health and mental state, but may also reduce the overall required therapy time and/or the prescription of further supporting medicaments. Thereby, the therapy outcome and efficacy will likely be increased. This ence may not only improve the physical state and quality of life of the patient, but may also reduce the overall treatment costs, which may generally be costly, e.g., for asthma patients.

Preferably, the obtaining of the measurement indicative of a disease status of a patient includes the performance of an actual or current measurement. For example, the measurement may be provided by measuring a physiological parameter or another parameter affecting the physiological state of the patient. For example, the measurement may be an actual measurement of a pollen concentration. In such case wherein an external parameter is obtained, alternatively, or in addition, the method may further include a determining of an actual location of the patient, wherein the obtaining of the measurement of a pollen concentration is specific for the patient's location. For example, the patient may carry a portable device with a coupled measurement module, e.g. a local weather server, configured to provide a pollen concentration measurement, such that the obtained measurement is specific for the current location of the patient, which may be more accurate than a general pollen concentration indicated for a particular region, e.g. by a central weather station or central server. Such measurements may be performed periodically and the patient may be prompted to query such measurements at predefined time points, so as to indicate a particular trend of the pollen concentration. By the same token, other measurements affecting the physiological state of the patient may be obtained, such as air humidity and/or air quality, e.g. for patients suffering from respiratory diseases.

Furthermore, the measurements may include the determining of an actual location of the patient, i.e. a geometrical position of the patient, such that particular regional measurements from e.g. a weather station or central server, e.g. of a pollen concentration, may be associated with the particular position of the patient, thereby increasing the accuracy of the measurement in the health risk and adherence evaluation performed by the evaluation unit.

Optionally, both an actual measurement and determining of the actual location of the patient are performed, so as to determine a trend and a more accurate exposure of the patient to the particular parameter of influence, e.g. by performing the evaluation in a time-dependent and position dependent manner. By the same token, such cumulative data may be averaged so as to reduce outliers and increase the validity of the measurements.

Preferably, the pollen concentration is also associated with at least one specific pollen or category of pollen. Thereby, the evaluation unit may differentiate between various pollen concentration measurements and may assess whether the obtained measurement is relevant for the respective patient. In other words, by obtaining the measurement as a measurement that is specific for one or more pollen, e.g. a mixture of pollen belonging to the same category or type of pollen, the evaluation unit may determine whether the measured pollen correspond to one or more allergens to which the patient may develop an allergic reaction or not. The signal may be accordingly adjusted.

The measurement indicative of a disease status of the patient, e.g. a measurement of a pollen concentration, may furthermore be obtained periodically and/or be based on a proximity of the location of the patient with a predefined location, wherein a probability of a potentially detrimental exposure to a particular parameter, e.g. an allergen, is determined based on the obtained measurement, preferably by evaluating the obtained measurement over a predefined period of time.

This ensures that the measurements may be provided at regular time points, such that the validity and accuracy of said measurements may be improved and exposure to e.g. particular pollen concentrations may be determined as predefined time points, so as to further assess the efficacy of an immunization treatment, for example. Furthermore, the measurement may be performed when a patient is in proximity of a location or region, wherein an increasing pollen concentration may occur or is known from previous measurements and/or actual measurements, e.g. from increasing symptoms of other patients at said location or from a central server or weather station. A proximity of the patient to such location may hence increase the exposure to particular allergens.

Although the inputted patient parameter at least comprises an indication of a performed treatment, e.g. an immunization treatment, the parameter may include further parameters or information, so as to improve the assessment of the adherence and/or a current health status or health risk of the patient. Accordingly, the inputted patient parameter may furthermore comprise the experiencing of one or more symptoms, a breathing difficulty, a swelling, a rash, a stress level, and/or a physical status. Accordingly, the inputted patient parameter may comprise disease specific parameters, such as, in addition to e.g. the performance of the immunization treatment, a type of symptoms, a level of symptoms, and/or a lack or relief of symptoms, and may furthermore comprise patient-specific parameters, such as a level of stress, an experienced level of support and/or motivation.

Such information may also be provided by means of the outputted signal to a physician, such that a physician may compare e.g. a current pollen prediction or measurement with one or more symptoms of the patient and/or may query the patient to input such information. Thereby, a medical platform may also inform a physician, when symptoms are not improving according to the pollen concentration. The physician may then optionally check and/or adjust the therapy and/or adjust the type and/or dose of a prescribed medicament or drug, e.g., when the symptoms are not experienced in line with a level of allergens for which the patient is currently treated.

In addition, personal parameters may be assessed, such as a patient's fear of side effects, its obliviousness, knowledge of the therapy, and/or confidence in the therapy, wherein such personal parameters may either be directly inputted by the patient or may be retrieved by prompting the patient a questionnaire to determine one or more of the above personal parameters. These personal parameters may furthermore be correlated with a medical capacity and support activity in order to assess whether the patient's personal experience potentially matches a lack of medical support, e.g. due to a lack of medicament resources or appointment possibilities, and to compare the personal parameters with an objective measure so as to determine a potential severity or seriousness of the personal parameters.

The inputted parameters are then processed by the evaluation unit and are each weighted or factored according to preset values or scales so as to determine a total adherence level. For example, disease specific and patient-specific parameters may each be weighted up to a percentage of between 30 and 50 percent of the total adherence, such that they may comprise an overall percentage of between 60 and 100 percent of the total adherence and may hence form the main parameters for determining the compliance course.

An example of various optional parameters and a corresponding factoring and weighting is provided in the table below:

| Patient | Medical practice | Source | Frequency |
|---------|------------------|--------|-----------|
| **Disease-specific (weighting 40%)** | | | |
| Seriousness of Symptoms | | Query | Periodically during occurrence of potential factors influencing disease |
| Loss of symptoms | | Query | Periodically during occurrence of potential factors influencing disease |
| | Therapy course Number of medicaments / therapy years | System | Update upon patient visit |
| | Therapeutic duration | System | Update upon patient visit |
| **Therapy-specific (weighting 5%)** | | | |
| | Complexity | System | Update upon patient visit |
| | Duration and frequency (perennial, pre-seasonal, short-term) | System | Update upon patient visit |
| **Medical support (weighting 10%)** | | | |
| Informing patient | | Query | Once per therapeutic year |
| System capacity or availability | | System | Update upon patient visit |
| Trust in physician and practice | | Query | Once per therapeutic year |
| **Socio-economical (weighting 5%)** | | | |
| | Age | System | Update upon patient visit |
| Support environment | | Query | Once per therapeutic year |
| **Patient-specific (weighting 40%)** | | | |
| Stress | | Query | Once per therapeutic year |

(continued)

| Patient-specific (weighting 40%) | | | |
|---|---|---|---|
| Motivation | | Query | Once per therapeutic year |
| Fear of side effects | | Query | Periodically during occurrence of potential factors influencing disease |
| | Obliviousness | System | Update upon patient visit |
| Knowledge of therapy | | Query | Once per therapeutic year |
| Faith in therapy | | Query | Once per therapeutic year |

Accordingly, various parameters may be factored differently and may furthermore be provided either at a patient's interface, e.g. at a remote device, or may be inputted into and retrieved from a system. These parameters may then each be scored so as to obtain a corresponding value for each class associated with the adherence, e.g. according to following formula:

$$A(n) = \frac{v_1 \cdot (\%) + v_2 \cdot (\%) + v_n \cdot (\%)}{n}$$

wherein the adherence (A) for each class is determined based on the individual variable (v) and a corresponding weighting and/or scoring for said variable, wherein each variable may be scored e.g. with a value ranging between 0 and 1 or between -10 and 10. Furthermore, for each adherence class a corresponding weighting is performed so as to provide a total adherence level, such that the risk of a patient remaining or losing adherence may be determined, e.g. according to the following formula:

$$A_{total} = \frac{A_1 \cdot (\%) + A_2 \cdot (\%) + A_n \cdot (\%)}{n}$$

wherein each adherence class (A) is factored according to the associated weighting percentage to define a total adherence level. The resulting adherence level or risk of losing adherence may then be output as a signal to a physician, wherein each of the adherence classes and the total adherence may be indicated with a corresponding color scaling corresponding with a risk of negatively impacting the adherence of the patient and hence a total risk of adherence loss of the patient.

For example, the color scaling may be provided as a gradient between red and green, wherein red indicates a corresponding high risk factor and green indicates a low risk factor or full compliance, wherein e.g. yellow or orange may furthermore indicated a potential problem. These indicated risks may continuously or periodically be evaluated to correlate actual adherence losses with the indicated risks and/or color scales. Such evaluation may be performed externally, e.g. at a backend implementing service or may be manually adjusted by a physician, e.g. based on patient information. In particular, such evaluation may be requested, when large deviations between e.g. the color indications and actual adherences are determined.

In order to further improve the adherence or compliance and reduce the risk of a factor such as an increasing pollen concentration adversely affecting the patient's health, the signal may furthermore comprise an indication of a required treatment. Thereby, a physician may not only be alerted about a current discrepancy with a predefined compliance course and e.g. a potential exposure to one or more allergens, but may furthermore inform and encourage a patient to perform a required treatment, e.g. an immunization treatment, and/or to seek medical assistance. The indication may also comprise a reminder to an upcoming and scheduled treatment and/or medical consultation, so as to emphasize the importance of said treatment in order to achieve the desired therapeutic effect.

The determined compliance course may furthermore be transmitted to a remote device or a remote monitoring system, wherein the determined compliance course is preferably automatically transmitted, when the threshold is exceeded.

Such remote device may e.g. be a portable device of a medical professional, e.g. in the form of a mobile phone, laptop computer, or tablet, or a medical assistance device configured to be carried by a medical professional. Alternatively, the remote device may be a stationary device, such as a computer system communicatively coupled to a database system. Preferably, the remote device is configured as or is part of a remote monitoring system, so as to providing a monitoring of the patient's adherence and, in particular, a health status of the patient. The transmission of the determined compliance course may e.g. be associated with the patient, either via corresponding information contained in the transmitted data or by a corresponding key that matches specific patient information stored in the remote device.

The determined compliance course may then be compared with a predefined compliance course stored in or otherwise provided to the remote device or monitoring system, which is specific for the therapeutic state of the patient. The comparison may e.g. be a comparison between for one or more time points, wherein a deviation from an optimal or previous compliance value is determined. Accordingly, a predefined compliance course may also be provided merely by such deviation, such that the monitoring of the adherence or the risk of adherence loss is essentially provided by such deviation. Accordingly, while the compliance values and levels may vary over time and be scored and factored according to actual values, the physician may be provided with an indication whether the patient is continuing the therapy, i.e. remains adherent, or is at risk of discontinuing the therapy, i.e. becoming not adherent.

Thereby, a user or medical professional may assess the current compliance level of the respective patient and an indication of a necessary medical intervention may be output or an alarm may be induced, e.g. a warning signal to the physician indicating that a patient needs to be contacted, even if a deviation of the determined compliance course from the predefined compliance course does not exceed a (patient-specific) threshold. Such warning signal may e.g. be required based on other patient-specific data that is provided to the remote device and is not available at a portable device of the patient, such as e.g. physiological values, and other parameters, as described in the above.

The transmission of the predetermined adherence course may occur via known standard communication protocols such as WLAN, LAN, WAN, Internet, Bluetooth, Bluetooth Low Energy, RFID, NFC, Zigbee, and/or via a cellular network, wherein one or more hubs or access points may be provided to perform a data hopping from e.g. a portable device of the patient to an end remote monitoring system.

Preferably, the transmission requires an authorization of the patient, wherein the authorization preferably requires the inputting of a patient-specific identifier. Such patient identifier or authorization may also be provided in advance so as to facilitate an automatic transmission. For example, the authorization may be provided by means of a portable token, e.g. via NFC, RFID, or Bluetooth, or may be provided by inputting a password into a portable device via a corresponding interface, e.g. as a voice command, a typographical password, a face scan, or fingerprint scan. The information may furthermore be inputted at a central system or computer in a physician's practice, wherein a physician may be required to input a password to access and modify the patient-specific data.

Furthermore, said compliance course may be stored, among other patient-specific data, in a cloud-based server or database, such that the determined compliance course may be retrieved at an arbitrary time-point and is hence generally available at any time-point and independent of a current network or transmission capacity or coverage. The remote device or monitoring system may require a coupling with the cloud-based server prior to the retrieval of the determined compliance course, so as to ensure that the patient-specific data is not retrievable by an arbitrary third party.

According to a further aspect of the invention, a portable device for monitoring an adherence of a patient is suggested, which comprises an interface for inputting at least one patient parameter, wherein said parameter comprises an indication of a performed treatment, an evaluation unit in communication with the interface and configured to determine a compliance course by evaluating an inputted parameter over a predefined period of time and comparing the determined compliance course with a predefined compliance course, and a means for obtaining at least one measurement indicative of a disease status of the patient, preferably of a pollen concentration, and being in communication with the evaluation unit. The device is furthermore configured to output a signal, if a deviation of the determined compliance course from the predefined compliance course exceeds a threshold and/or the measurement indicative of a disease status of the patient exceeds a predefined tolerance limit.

The portable device may hence be configured to perform the method as described in the above, such that like elements are not repeatedly described in further detail. The portable device may e.g. be a mobile phone, PDA, tablet, or notebook and comprise an implemented module, e.g. a CPU-based module or an implemented interactive multimedia content file. Furthermore, one or more functions may be implemented by a corresponding application in the form of computer readable code stored on a computer readable medium contained in the portable device.

The interface of the portable device may e.g. be comprised of a keypad or keyboard, which may also be implemented as a touch-screen function of a display of the portable device, e.g. on a mobile phone or smart phone. By providing a display, a user or patient may easily grasp the presented information and the interrelation of various features. Furthermore, a display may be used to output the signal in the form of a visual indicator, e.g. by outputting a graphical representation of a determined health risk as a function of the combined deviation, threshold, pollen concentration and tolerance limit. Such signal may e.g. be provided by a device configured for medical personnel or a physician whereas a portable device configured for a patient may only indicate or request e.g. an increase of the frequency of the inputting of the patient parameters and/or a contacting of the physician.

Furthermore, the evaluation unit, which is communicatively coupled with the interface may be part of a control unit or control logic, which may be implemented e.g. in the form of a CPU coupled to a memory and data storage. The evaluation unit may hence be part of the normal functioning of the device and may be configured to control various shared features, such as a display and/or audio function of the portable device.

The means for obtaining a measurement indicative of a disease status of the patient, e.g. of a pollen concentration, may be provided as a central measurement retrieved from a central server or weather station.

Accordingly, the portable device may be implemented with a wireless communication module configured to communicate with a remote device or to receive data containing information regarding the measurement of e.g. a pollen concentration and being transmitted or broadcasted to the portable device. Such wireless communication module may e.g. be configured to communicate via standardized communication protocols as described in the above, e.g. via WLAN, LAN, WAN, Bluetooth, Bluetooth Low Energy, RFID, NFC, Zigbee, and/or via a cellular network. The wireless communication module may furthermore be configured to perform a bi-directional communication, such that data may also be transmitted to and exchanged with a remote device or monitoring system.

The implementation on a portable device furthermore has the advantage that a patient may be provided with additional information during the treatments and there between and furthermore in addition to the outputted signal. Accordingly, further information may be communicated to the patient even in the absence of a consultation by a medical professional.

For example, the portable device may output supportive information for the user or patient, so as to inform the patient about the prescribed therapy and the current status of the therapy. The portable device may e.g. output a therapy course, a symptom graph or course, preferably combined and correlated with a pollen concentration trend, background information regarding the therapy and/or the underlying disease, e.g. the specific allergy of the patient, developments, news, and/or a pollen trend prediction, preferably for a particular region, e.g. Europe. Thereby, a patient is better informed, which may further improve the compliance level. The information may furthermore be provided in an interactive manner, such that the user or patient may retrieve particular information and may request further information and/or contact a medical professional regarding a particular topic.

At a portable device configured for a physician, detailed information regarding the determined compliance may furthermore be outputted, wherein each factor influencing the determined compliance is outputted and provided with e.g. a key or coloring to indicate a deviation from a desired level of each factor, as described in the above. For example, the factors or parameters may be divided into particular categories, such as patient-specific parameters and disease-specific parameters, and may be further subdivided to increase the level of detail. Each of the factors may then be indicated according to a color scheme, wherein e.g. a green color indicates that the factor is not problematic and red indicates that a problem may exist. By the same token, yellow or orange coloring may indicate that the factor deviates from an optimal or desired value and may require attention of the physician, so as to improve the adherence. Problematic factors may furthermore be provided together with a solution or an indication for the medical professional to get in contact with the patient for further medical assistance

The provision of a monitoring in the form of a portable device for a patient hence has many advantages, which include, aside from a higher level independency, an increase in transparency of the ongoing therapy and the usage of patient-specific data. Furthermore, the portable device may be a device which is regularly used by the patient or physician, such as a smart phone, such that both a monitoring and the provision of information may be provided at any time point and independent of the location of the patient or physician. For example, the monitoring may be provided essentially on a real-time basis, which may significantly increase the safety of the patient and the efficacy of the therapy, in particular in view of static and periodic consultations with a medical professional or physician. Together, this may significantly increase the compliance as the patient may be reminded of e.g. the relevancy of the treatments, thereby ensuring that the patient maintains adherent.

As described in the above, the means for obtaining a measurement indicative of a disease status of the patient may furthermore be configured as a measurement device coupled to the device for performing an actual measurement and/or the device may furthermore comprise a localization module for determining an actual location of the patient, wherein the evaluation unit associates the measurement to the patient's specific location, wherein the measurement device and evaluation unit are preferably configured to associate a measurement of a pollen concentration with at least one specific pollen or category of pollen.

Preferably, the portable device may be coupled to a sensor, which in the case of an immunization treatment or therapy may be a sensor for measuring a pollen concentration. Such sensor and measurement may e.g. be provided by e.g. a weather station, for example, via a central server, or may be provided by means of one or more evaluated actual measurements of pollen concentration received from a central service, such as a pollen warning service, e.g. in Austria, or a pollen information service, e.g. in Germany. The portable device may furthermore comprise a GPS-module in order to determine an actual location of the patient, so as to determine a trend and a more accurate exposure of the patient to e.g. the particular pollen concentrations, e.g. by performing the evaluation in a time-dependent and position dependent manner. To further increase the accuracy of the localization of the patient, the portable device may be configured to determine its position based on position information and signal strengths from a plurality of access points or nodes that are in communication and in proximity of the patient, e.g. from a plurality of base stations or nodes, combined with retrieved mapping data. The localization may be performed by a network, so as to save energy and computation capacity on the portable device, but may also be performed on the portable device itself, so as to increase the level of privacy.

Preferably, the means for obtaining at least one measurement indicative of a disease status of the patient, e.g. of a pollen concentration, is configured to obtain said measurement periodically and/or based on a proximity of the location of the patient with a predefined location, wherein the evaluation unit is configured to determine a probability of a potentially detrimental exposure to a particular parameter, e.g. an allergen, based on the obtained measurement, preferably by evaluating the obtained measurement over a predefined period of time.

For example, an actual pollen concentration measurement may be retrieved from one or more coupled pollen sensors, when the portable device determines that the patient is in proximity of a location or region, wherein an increasing pollen concentration may occur or is known from previous measurements and/or actual measurements, e.g. from a central server or weather station. In other words, the portable device may store information regarding actual pollen concentrations or a trend prediction of pollen concentrations for a particular region, e.g. in the form of a pollen map, and may actively compare a determined location or position with said region, so as to assess whether the patient may be exposed to an allergen due to a potentially increased pollen concentration in the atmosphere. A proximity of the patient to such location may hence increase the exposure to particular allergens, wherein an automatic measurement may evaluate whether this is actually the case.

In order to further increase the safety of the patient, the device may furthermore be configured to output an indication of a required treatment, e.g. an immunization treatment, as a part of the signal, preferably on a display of the device.

Thereby, a physician may not only be alerted about a current discrepancy with a predefined compliance course and a potential exposure to e.g. one or more allergens, but may furthermore encourage a patient to perform a required treatment and/or to seek medical assistance. The indication may also comprise a reminder to an upcoming and scheduled treatment, so as to emphasize the importance of said treatment in order to achieve the desired therapeutic effect. The indication may also require a confirmation of the patient and may furthermore include therapy information, e.g. by outputting the determined compliance course and by indicating the positive effect of a particular compliance range so as to achieve the desired therapeutic effect. By the same token, the therapy course may be outputted indicating a progress or remainder of the therapy. Furthermore, the indication may emphasize the necessity and/or advantages of the therapy so as to overcome any potentially existing doubts. Accordingly, such information may further improve the adherence of the patient, e.g. by supporting or motivating the patient to continue the therapy and to finish their treatment.

To enable the portable device to communicate with other devices and/or a server, the device may furthermore comprise a wireless communication module that is in communication with the evaluation unit. The evaluation unit may be configured to transmit the determined compliance course to a remote device or a remote monitoring system via the wireless communication module, e.g. a cell phone or mobile terminal, wherein the evaluation unit is preferably configured to automatically transmit the determined adherence course, when the threshold is exceeded.

Accordingly, the portable device may be in communication with a remote monitoring device, e.g. a backend monitoring device operated by a medical professional or physician. By transmitting the determined compliance course, in particular when the threshold is exceeded, a physician may be informed about a declining compliance and a potential health risk or risk of adherence loss of the patient.

The data may be transmitted anonymously, so as to provide a level of privacy of the patient data, but is preferably transmitted with a patient identifier, such that the physician may associate the determined compliance course with a particular patient. The physician may then intervene, e.g. by establishing a communication with the patient, so as to ensure that a predefined compliance course is re-established or a determined deviation from the previous compliance is reduced. Furthermore, the physician may retrieve further patient data, e.g. personal data from previous consultations, from a database coupled to the remote monitoring device so as to determine an appropriate manner of intervention and/or communication with the identified patient.

Preferably, the evaluation unit is configured to receive a patient-specific identifier and to transmit the determined compliance course after receiving an authorization provided by said patient-specific identifier. As described in the above, such authorization may be provided e.g. by means of a portable token, e.g. via NFC, RFID, or Bluetooth, or may be provided by inputting a password into a portable device via a corresponding interface, e.g. as a voice command, a typographical password, a face scan, or fingerprint scan. The patient may furthermore configure the evaluation unit and/or the wireless communication module to automatically couple the portable device with a specific remote device, such that the authorization may be enabled in advance so as to facilitate an automatic transmission. A portable device configured for a physician may not require an authorization of the patient, but may be authorized e.g. by a voice command or typographical password to access and modify the patient data.

Still further, according to another aspect of the invention, a system for monitoring an adherence of a patient is suggested, comprising a portable device as described in the above and a remote monitoring device comprising a database system storing patient data and configured to be in communication with the portable device for receiving a determined compliance course, wherein the remote monitoring device is configured to compare the determined compliance course with a predefined compliance course. The predefined compliance course may be based on the stored patient data, and the remote monitoring device is furthermore configured to obtain at least one measurement indicative of a disease status of the patient, preferably a measurement of a pollen concentration, and to output a signal to the portable device, if a deviation of the determined compliance course from the predefined compliance course exceeds a threshold and/or the measurement indicative of a disease status of the patient exceeds a predefined tolerance limit.

The remote monitoring device may be configured as a computer system and may enable the various functions at least in part, preferably entirely, via a web-based implementation. The database system or data storage as well as the computation logic may be implemented either locally or via a cloud-based implementation, e.g. an ISO/IEC certified cloud storage, which is communicatively coupled to the remote monitoring device. Data is stored according to common security standards and external data, e.g. to provide the measurements indicative of a disease status of the patient, may be provided via secured application programming interfaces.

The patient-specific data and login data may furthermore be stored with data encryption and may be secured in the database system via e.g. a two-step firewall, encrypted VPN, and/or a multi-step security and access control system. By the same token, backups may be regularly established using the same level of security. The data may be processed anonymously by removal of any associations between the patient and the remote monitoring system or medical practice and may furthermore be processed with Hash-values, such that in the case of data abuse or unauthorized access or manipulation of the data no patient-specific or sensitive data may be retrieved.

Accordingly, the remote monitoring device provides a secured and readily available platform for performing an adherence monitoring and which is communicatively coupled to at least one portable device of a patient or physician so as to form a monitoring system. The platform may comprise various features, including a dashboard, a patient management system storing e.g. personal patient data, adherence data, compliance courses, therapy, therapy courses, calendars and notes, appointments or consultations, availability, and communication protocols related to appointments or consultations. Furthermore, the platform may comprise various data related to the respective medical practice.

Thereby, the remote monitoring system enables that medical professionals may be provided with an overview of one or more patients and may assess the health and adherence status of each individual patient, so as to perform a medical intervention or initiate a communication with the patient, e.g. when the patient's health is at risk due to a declining compliance and an inclining pollen concentration. Furthermore, by coupling the data obtained from the portable device with patient-specific data stored in the database, a more accurate health assessment may be obtained, e.g. by inputting a particular type of immunization treatment or specimen and/or physiological conditions.

The remote monitoring device is further preferably configured to provide a bi-directional communication with the portable device and to transmit a request of increasing the parameter inputting frequency and/or an indication of a required treatment as a part of the signal to the portable device and/or a second remote device.

Due to the bi-directional communication, the remote monitoring device and the portable device are coupled and may exchange information or data in either direction. Thereby, a patient may e.g. be provided with detailed information regarding his or her therapy and may hence be supported during a prescribed therapy and specifically in adequately performing treatments, even in the absence of a personal consultation by a physician or a medical professional.

By the same token, physicians may be facilitated in performing a monitoring of the adherence of a plurality of patients by receiving updated information and determined compliance courses and to accordingly determine whether a medical intervention or alerting of a patient may be required. The second remote device may e.g. be configured as a portable medical assist device, such that a medical professional carrying said second remote device may be accordingly alerted.

## Brief description of the drawings

The present disclosure will be more readily appreciated by reference to the following detailed description when being considered in connection with the accompanying drawings in which:

Figure 1 is a schematic view of a compliance course of a patient in view of a varying pollen concentration;

Figure 2 is a schematic view of a weighting of various factors to determine an adherence;

Figure 3 is a schematic view of a logic implementation of an evaluation unit to perform a monitoring of a patient adherence;

Figure 4 is a schematic view of a portable device for monitoring an adherence of a patient in a system; and

Figure 5 is a schematic view of a system comprising a portable device and a remote monitoring device using a cloud-based encryption.

Detailed description of preferred embodiments

In the following, the invention will be explained in more detail with reference to the accompanying figures. In the Figures, like elements are denoted by identical reference numerals and repeated description thereof may be omitted in order to avoid redundancies.

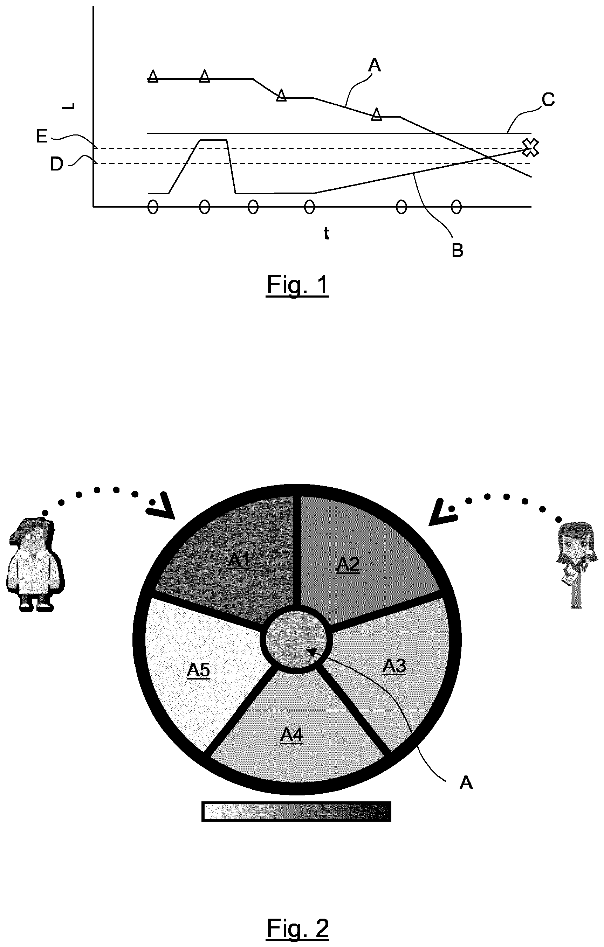

In Figure 1 the interrelationship between the performed immunization treatment (triangular elements) and a prescribed therapy (circular elements) is schematically depicted over time (t). Accordingly, a number of immunization treatments may be required and are prescribed for particular time points, e.g. a specific date, so as to provide a predefined interval between said immunization treatments. The immunization treatment may be performed e.g. by means of a syringe or loaded needle tip to perform a subcutaneous immunotherapy (SCIT), i.e. provide an injection of a medicament or low dose of a (modified) pollen allergen concentration in order to perform a hyposensibilization of the patient for a particular allergen. The immunization treatment may be performed at a medical facility, e.g. at a physician's office or practice, or at a remote location, e.g. at a patient's home via self-administration, such as in the form of tablets or drops to perform an oral treatment or a sublingual immunotherapy (SLIT), or epicutaneously.

As indicated by the circular elements, the therapy may require that the individual immunization treatments are performed at particular intervals. However, this does not necessarily mean that said treatments are indeed performed at these time-points, since a patient may not be capable of performing the treatment, may be reluctant to the therapy, or does not prioritize said treatments. Accordingly, the treatments may be performed at arbitrary time-points, as indicated by the triangular elements. Thereby, compliance to the immunotherapy may vary. Such compliance is hence an indication whether a patient performs the number of immunization treatments according to the prescription or not.

The compliance of the patient may be determined, e.g. by an evaluation unit, based on a deviation between the prescribed time-point and the actual time-point of the immunization treatment and may be evaluated over time and for a number of prescribed time-points, so as to determine a compliance course, which is depicted in Figure 1 by "A". According to the example, a patient performs the first two immunization treatments in exactly the prescribed time-frame, which is depicted by the matching time-point of the triangular and circular elements along the time-line "t". Accordingly, disregarding potential other patient-specific factors, the compliance may be determined to be at an optima level, denoted by "L", indicating that the patient is adherent.

However, at the third prescribed time-point no immunization treatment is performed, for example, since the patient is indisposed, does not have the required treatment, e.g. when self-medication is performed, or has forgotten a corresponding appointment or consultation with a physician. Instead, said immunization treatment is performed with a delay, i.e. between the third and fourth prescribed immunization treatment. As a consequence, the determined compliance declines to a lower level until the immunization treatment is administered. By the same token, a longer delay between the performed immunization treatment and the prescribed time-interval is provided for the fourth prescribed immunization treatment. Even further, the fifth and sixth immunization treatments have been omitted. Therefore, the determined compliance course further declines, as indicated in Figure 1.

At the same time, a physician may predefine a level of compliance, based e.g. on patient-specific parameters, a frequency of immunization treatments and a complexity of the disease or allergy. A corresponding predefined compliance course may hence be at a lower level compared with full compliance, as full compliance may not always be required and may put a burden on the patient, which may lead to a lower acceptance of the immunotherapy. Such a predefined compliance course is indicated by "C". As indicated in Figure 1, the first delay and corresponding decline of the compliance may not result in a compliance level that drops below the predefined compliance course "C". However, the repeated delay may result in a determined compliance course "A" being lower than the predefined compliance course "C", as indicated by the intersection of lines "A" and "C" in Figure 1. A further delay and omission of immunization treatments may furthermore result in a decline of the determined compliance level below the predefined compliance course to a level that exceeds a predefined threshold, indicated with the dashed line "D" in Figure 1.

Although such low compliance level may result in a reduced efficacy of the therapy and is generally undesirable, this may not immediately trigger a signal to the patient. For example, although the patient may be motivated to continue the immunotherapy, a warning signal may not be immediately required in a situation wherein only low concentrations of pollen are present, e.g. during winter. Such pollen concentration measurement is indicated by line "B". Nevertheless, a signal may be output since the dosage of the immunization treatments may have to be accordingly adjusted. Between the first and third prescribed immunization treatment a steep increase in the pollen concentration is provided between the first and third prescribed immunization treatment, as indicated by the incline of the level "L" for line "B". However, in this case the determined compliance of the patient follows an optimal compliance course, such that the patient is determined to be adherent and the immunization of the patient may be at a sufficient level to avoid any adversary reactions due to the increase of the pollen concentration.

In contrast, the continuous increase of the pollen concentration after the fourth prescribed time-point, although ultimately reaching the same level of pollen concentration after the sixth prescribed time-point, may result in an increased health risk of the patient due to the simultaneous decline in the determined compliance course, which are caused by the delayed and omitted immunization treatments. In this case, the exceeding of the lower threshold limit of the compliance and the exceeding of the upper tolerance limit "E" of the pollen concentration may pose a risk to the patient, i.e. a risk of experiencing an allergic reaction during the therapy, which is indicated in Figure 1 by the cross element. Therefore, a signal or warning signal may alert the patient that the administration of a medicament may be required and may simultaneously be reminded of the importance of reestablishing the desired adherence to achieve the required efficacy of the immunotherapy, e.g. by prompting that the frequency of the inputting of the performed treatments and patient parameters are increased.

In the exemplary embodiment according to Figure 1, the compliance course is primarily determined based on the performance of the prescribed immunization treatment and a potential temporal discrepancy of the actual performance with the predefined time-point. In addition to the inputting of a performed immunization treatment, additional patient parameters may be inputted, which may be factored into the determining of the overall adherence, as shown in Figure 2. Accordingly, the adherence may be determined based on various factors A1-A5, which may be associated with patient-specific parameters, disease-specific parameters, or parameters related to the medical facility, as indicated by the arrowheads. The importance of each factor may furthermore be weighted in assessing the overall adherence, in particular when determining the compliance course or trend and/or assessing the probability of a change in said course. Such weighting is schematically depicted by the sizes of each factor in the form of a pie chart, wherein the coloring indicates the impact on the determined adherence or total compliance level, e.g., wherein a bright color indicates a low compliance and a darker color indicates a higher compliance. For example, the disease specific and patient-specific parameters may each be weighted up to a percentage of between 30 and 50 percent of the total adherence, such that they may comprise an overall percentage of between 60 and 100 percent of the total adherence and may hence form the main parameters for determining the compliance course. Such graphical depiction may furthermore be included in the signal, such that the attention of a physician may be drawn to those factors influencing the adherence most.

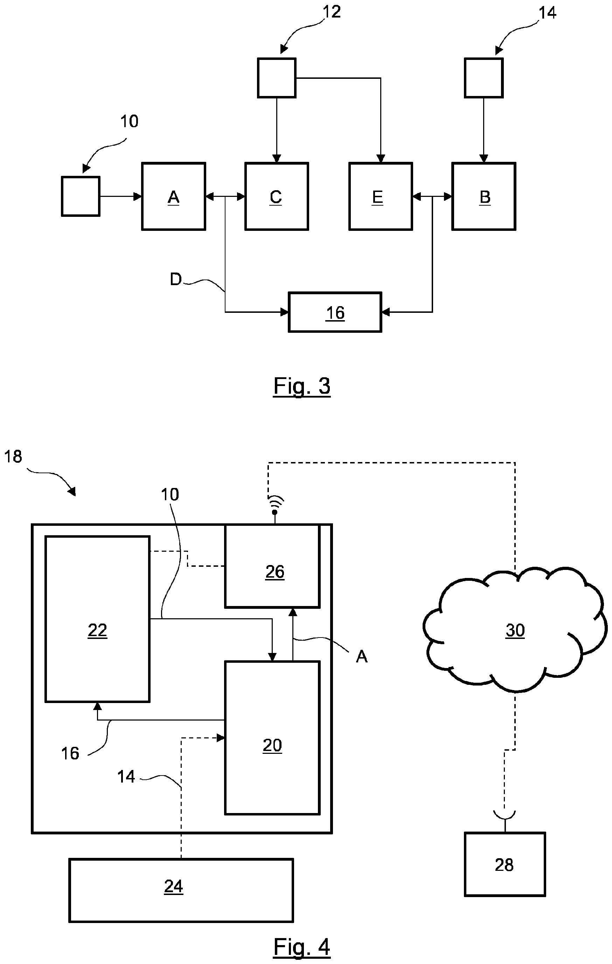

In Figure 3 a logic implementation of an evaluation unit is schematically depicted which is configured to perform a monitoring of a patient adherence, similar to the exemplary embodiment according to Figure 1. Accordingly, a compliance course "A" may be determined based on an inputted indication of a performed immunization treatment 10, which may be evaluated over time so as to include a number of inputted immunization treatments. Furthermore, the compliance course may be determined based on a regularity of an interval between a number of immunization treatments, e.g. when a prescribed therapy requires a constant interval between each pair of immunization treatments.

The evaluation unit may hence determine the compliance course based on temporal deviations from a fixed interval, e.g. via a clock or timer provided on a CPU or calendar, for example. Alternatively, the compliance course may be determined based on the inputted indication of a performed immunization treatment 10 and one or more patient parameters, e.g. patient-specific parameters 12, which may be stored in the evaluation unit or may be provided via an interface, such as a wireless communication module or a synchronization with a data server via a corresponding communicative coupling.

The one or more patient-specific parameters may furthermore be used to provide a predefined compliance course "C", which may take into consideration that an optimal compliance, although this would provide the highest therapeutic efficacy, is not required and may not be aimed at in order to avoid any adversary influence on the patient, such as a reduced acceptance of the therapy. The determined compliance course "A" may then be compared with the predefined compliance course "C" and may result in a deviation. Generally, the evaluation unit may also derive a deviation from the determined compliance course "A" itself, such that a predefined compliance course "C" may not be required or may be replaced by one or more deviation calculations. The evaluation unit may use said deviation as a measure whether the determined compliance course "A" falls below a particular level, which may be adversary for the patient by comparing said deviation with a threshold "D". Such deviation may hence result in an absolute deviation or an absolute compliance level, which may potentially pose a risk to the patient. Accordingly, such risk may be indicated when the deviation exceeds a threshold "D", which may be stored in the evaluation unit, e.g. in a data storage or memory, and which may indicate that a patient is no longer adherent.

The evaluation unit may then output a signal 16, e.g. a warning signal, if the deviation exceeds such threshold "D", e.g. when an absolute deviation is considered too large. However, the evaluation unit preferably also factors in or uses a pollen concentration measurement 14 in its calculation, which may be evaluated so as to provide an average pollen concentration "B", excluding any potential outliers and having an improved validity. The average pollen concentration "B" may be provided e.g. via a communicative coupling with a weather station, which may transmit location specific or regional actual pollen concentration measurements as well as pollen concentration trends and predictions. The evaluation unit may then compare the average pollen concentration "B" with a patient-specific tolerance limit "E", which may also be stored in the evaluation unit, e.g. on a data storage, and may be derived from the one or more patient-specific parameters 12.

Thereby, the evaluation unit not only processes the adherence data, but also considers the current or future pollen concentrations that may be relevant for the patient and may induce an allergic reaction. Based on a comparison with the patient-specific tolerance limit "E" the relevancy for the particular patient may be assessed. For example, a physician may be informed that low levels of pollen concentrations are present that are only related to a particular allergen and may not severely affect the patient's health, e.g. via the signal 16. However, although such information may be provided, the physician is primarily informed about the adherence of the patient. Should the patient be e.g. not adherent, a physician may be informed that a therapy may be ineffective. Accordingly, when high levels of pollen concentrations are present that adversely affect the patient and may trigger an immune reaction, the physician may hence obtain this information via the outputted signal 16, which may comprise a warning that the patient may not be adherent and may comprise a low level of immunization, such that the patient may have an increased level of susceptibility.

In the embodiment according to Figure 4, a portable device 18 for monitoring an adherence of a patient is schematically depicted in a system comprising a cloud-based database 30 and a remote monitoring device 28.

The portable device 18 according to the embodiment is provided as a smart phone, wherein an interface 22 for inputting information into the smart phone is provided as a display 22 formed as a touch-screen, such that information may be inputted into the smart phone by touching a particular region of the display 22, e.g. by touching a graphical representation of a keypad or keyboard. The smart phone is furthermore configured to perform the method as described in the above by implementation of an evaluation unit 20, which may be part of a control unit and/or processing unit with an on-board memory and storage device, e.g. as a CPU and a coupled flash memory.

Accordingly, the evaluation unit 22 is configured to receive one or more indications of an immunization treatment 10 via the interface 22 and may determine a compliance course "A" based on said indications, e.g. by comparing the indications with data indicative of a prescribed therapy, which are stored in the evaluation unit 20. In addition, the evaluation unit 20 is configured to receive a pollen concentration measurement 14 via a pollen sensor 24, which is coupled to the smart phone and is communicatively coupled with the evaluation unit 20 via a respective interface. For example, the pollen sensor 24 may be provided by a weather station and may be coupled to the evaluation unit 20 via RFID. Alternatively, the one or more measurements may have been obtained via downloading using a hardware coupling with e.g. a computer system.

Based on the actual pollen concentration measurement 14 or a trend in the pollen concentration and the determined compliance course "A", the evaluation unit 20 may output a signal 16, as described in the above in view of e.g. Figures 1 and 2, e.g. in the form of a warning signal to a physician. Hence, the evaluation unit 20 may compare the determined with a predefined compliance course and output a signal 16, if a deviation of the determined compliance course "A" from the predefined compliance course exceeds a threshold and/or the measurement of a pollen concentration 14 exceeds a predefined tolerance limit.

The signal 16 may be output to the physician via the display 22 in the form of a visual indicator, which may e.g. indicate a rising pollen concentration and a decline in the immunization efficacy of the patient. Furthermore, such signal 16 may include an indirect reference to guidelines or best practices, such that the physician may find an indication of a medicament or drug to be used in case the patient develops symptoms associated with an allergic reaction. Furthermore, on a patient device, the signal 16 may prompt the patient to continue inputting the parameters and/or seek medical assistance in order to continue the immunotherapy, so as to improve the adherence and the efficacy of the therapy.

In order to provide a monitoring of the adherence of a patient in a remote monitoring device 28, the portable device 18 or smart phone furthermore comprises a wireless communication module 26, which may be configured to provide a bi-directional communication upon coupling with a remote device and may hence be configured as a transceiver. The wireless communication module 26 is in communication with the evaluation unit 20, such that the evaluation unit may transmit the determined compliance course "A" to a remote monitoring device 28 via the wireless communication module 26. The remote monitoring device 28 may receive the determined compliance course "A", and optionally further patient-related data, via a database or storage that is cloud-based, as indicated by reference numeral 30. Accordingly, the coupling between the portable device 18 and the remote monitoring device 28 may be provided indirectly via said cloud-based database 30. However, such interface is optional. By providing the determined compliance course "A" to the remote monitoring device 28 a physician or medical professional is enabled to monitor the patient's health status for a plurality of patients and may perform a medical intervention or establish a communication with the portable device 18, if necessary.

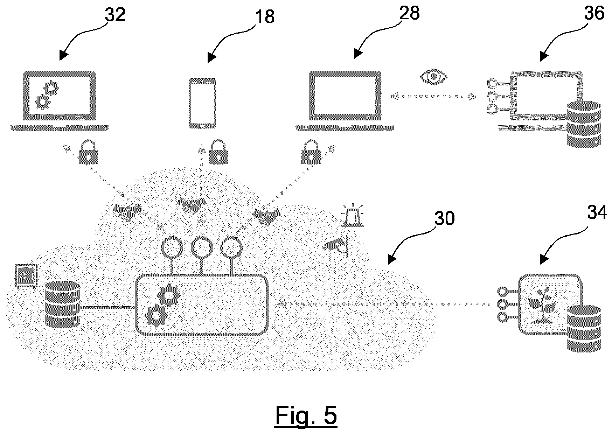

A system comprising a portable device 18 and a remote monitoring device 28 using a cloud-based encryption is depicted schematically in the embodiment according to Figure 5.

A computational logic 30 of the system as well as a database system or storage may be provided as a central element as indicated by reference numeral 30. Accordingly, a plurality of portable devices 18 as well as remote monitoring devices 28 may be communicatively coupled via a shared database 30, wherein information may be processed and encrypted and wherein data is secured via e.g. a two-step firewall, encrypted VPN, and/or a multi-step security and access control system, as indicated by the corresponding graphical icons. By the same token, backups may be regularly established using the same level of security, as indicated by the cylindrical symbols. Access to the database and computational logic 30 is monitored, so as to prevent data abuse or unauthorized access or manipulation of the data and to avoid that patient-specific or sensitive data may be retrieved by unauthorized third parties.

The system hence optionally uses a cloud-based implementation, wherein each of the remote monitoring devices 28 may access the required data and establish the required communication e.g. via a web-based implementation and wherein each portable device 18 may be provided with the required patient-specific information via an interactive multimedia content file or implemented application.

In addition, the system is optionally coupled with a weather station and pollen station 34, which may provide pollen concentration measurements, trends, and predictions for a particular location or region to the respective portable device 18.

Furthermore, the system comprises a backend system 32, which ensures that updates may be performed on the computational logic level and for implementations in the evaluation unit and background information may be circulated, as required. In addition, the remote monitoring devices 28 may optionally be in communication with a shared central server 36, which does not require encryption of information, such that the information may be freely shared. Such information may e.g. relate to current general developments in immune therapy, public health advice, newsletters, general pollen and allergy information, and/or information emphasizing the importance of immunotherapy.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

Drug safety/ pharmacovigilance reporting requirements

Legal reporting requirements of occurring adverse events/ drug reactions during the therapy as well as identified special situations e.g. pregnancy during the therapy, lack of drug effect after completion of therapy, overdose or medication error may need to be fulfilled. To enable the Marketing Authorization Holder (MAH) performing the risk assessment and risk management the name and contact data of the reporter and identification of the patient with initials and birth date, name of medicinal product, allergen composition, dosage with unit and strength and date of administration and occurrence as well as detailed description of adverse events or reactions may need to be reported to the MAH immediately after the adverse event/ drug reaction/ special situation occurred or the information was received from the patient. The patient may be informed about the sense of the reporting requirements.

List of reference numerals

| | |
|---|---|
| 10 | Indication of immunization treatment |
| 12 | Patient-specific parameter |
| 14 | Pollen concentration measurement |
| 16 | Signal or warning signal |
| 18 | Portable device |
| 20 | Evaluation unit |
| 22 | Display and interface |
| 24 | Pollen sensor |
| 26 | Wireless communication module |
| 28 | Remote monitoring device |
| 30 | Database |
| 32 | Backend system |
| 34 | Weather station and pollen station |
| 36 | Shared information server |
| A | Determined compliance course |
| A1-5 | Adherence factors |
| B | Averaged pollen concentration |
| C | Predefined compliance course |
| D | Threshold |
| E | Patient-specific tolerance limit |
| L | Level or percentage |
| t | Time |

**Claims**

1. Method for monitoring an adherence of a patient, comprising the steps of:

   - inputting at least one patient parameter into an evaluation unit, said parameter comprising an indication of a performed treatment (10);
   - determining a compliance course (A) by evaluating, in the evaluation unit (20), the inputted parameter over a predefined period of time and comparing the determined compliance course (A) with a predefined compliance course (C);
   - obtaining, by the evaluation unit (10), at least one measurement indicative of a disease status of the patient (14); and
   - outputting a signal (16), if a deviation of the determined compliance course (A) from the predefined compliance course (C) exceeds a threshold (D) and/or the measurement indicative of a disease status of the patient (14) exceeds a predefined tolerance limit (E).

2. Method according to claim 1, wherein the measurement indicative of a disease status of the patient (14) comprises a measurement of a physiological parameter and/or a measurement affecting the physiological state of the patient.

3. Method according to claim 1 or 2, wherein the performed treatment is an immunization treatment and wherein the measurement indicative of a disease status of the patient (14) comprises a measurement of a physiological parameter of the patient and/or a measurement of a pollen concentration, wherein the pollen concentration is preferably associated with at least one specific pollen or category of pollen.

4. Method according to any of the preceding claims, wherein the obtaining of the measurement indicative of a disease status of the patient (14) includes the performance of an actual measurement and/or wherein the method further includes determining an actual location of the patient, wherein the obtaining of the measurement indicative of a disease status of the patient (14) is specific for the patient's location.

5. Method according to claim 4, wherein the measurement indicative of a disease status of the patient (14) is obtained periodically and/or based on a proximity of the location of the patient with a predefined location, and wherein a probability of a potentially detrimental exposure to a particular parameter, preferably an allergen, is determined based on the obtained measurement, preferably by evaluating the obtained measurement over a predefined period of time.

6. Method according to any of the preceding claims, wherein the inputted patient parameter furthermore comprises at least one of: the experiencing of one or more symptoms, a breathing difficulty, a swelling, a rash, a stress level, and a physical status.

7. Method according to any of the preceding claims, wherein the signal (16) comprises an indication of a required immunization treatment.

8. Method according to any of the preceding claims, wherein the determined compliance course (A) is transmitted to a remote device (28) or a remote monitoring system, wherein the determined compliance course (A) is preferably automatically transmitted, when the threshold is exceeded.

9. Method according to claim 8, wherein the transmission requires an authorization of the patient, said authorization preferably requiring the inputting of a patient-specific identifier.

10. Portable device (18) for monitoring an adherence of a patient, comprising:

    - an interface (22) for inputting at least one patient parameter, said parameter comprising an indication of a performed treatment (10), preferably an immunization treatment,
    - an evaluation unit (20) in communication with the interface (10) and configured to determine a compliance course (A) by evaluating the inputted parameter over a predefined period of time and comparing the determined compliance course (A) with a predefined compliance course (C), and
    - a means for obtaining at least one measurement indicative of a disease status of the patient (24, 34), preferably of a pollen concentration, and being in communication with the evaluation unit (20),

wherein the device (18) is furthermore configured to output a signal (16), if a deviation of the determined compliance course (A) from the predefined compliance course (C) exceeds a threshold (D) and/or the measurement indicative of a disease status of the patient (14) exceeds a predefined tolerance limit (E).

**11.** Device (18) according to claim 10, wherein the means is configured as a measurement device (24) coupled to the device for performing an actual measurement (14) and/or wherein the device furthermore comprises a localization module for determining an actual location of the patient, wherein the evaluation unit associates the measurement (14) to the patient's specific location, wherein measurement device (24) and evaluation unit (20) are preferably configured to associate a pollen concentration measurement with at least one specific pollen or category of pollen.

**12.** Device (18) according to claim 11, wherein the means for obtaining at least one measurement indicative of a disease status of the patient (24, 34) is configured to obtain said measurement periodically and/or based on a proximity of the location of the patient with a predefined location, and wherein the evaluation unit (20) is configured to determine a probability of a potentially detrimental exposure to a particular parameter, preferably an allergen, based on the obtained measurement, preferably by evaluating the obtained measurement over a predefined period of time.

**13.** Device (18) according to any of claims 10 to 12, wherein the device (18) is furthermore configured to output an indication of a required treatment, preferably of an immunization treatment, as a part of the signal (16), preferably on a display (22) of the device (18).

**14.** Device (18) according to any of claims 10 to 13, further comprising a wireless communication module (26) being in communication with the evaluation unit (20), wherein the evaluation unit (20) is configured to transmit the determined compliance course (A) to a remote device (28) or a remote monitoring system via the wireless communication module (26), wherein the evaluation unit (20) is preferably configured to automatically transmit the determined compliance course (A), when the threshold is exceeded.

**15.** Device (18) according to claim 12, wherein the evaluation unit (20) is configured to receive a patient-specific identifier and to transmit the determined compliance course (A) after receiving an authorization provided by said patient-specific identifier.

**16.** System for monitoring an adherence of a patient, comprising:

- a portable device (18) according to any of the claims 10 to 15, and
- a remote monitoring device (28) comprising a database system (30) storing patient data and configured to be in communication with the portable device (18) for receiving a determined compliance course (A),

wherein the remote monitoring device (28) is configured to compare the determined compliance course (A) with a predefined compliance course (C), said predefined compliance course (C) being based on the stored patient data (12), and wherein the remote monitoring device (28) is furthermore configured to obtain at least one measurement indicative of a disease status of the patient (14), preferably of a pollen concentration, and to output a signal (16) to the portable device (18), if a deviation of the determined compliance course (A) from the predefined compliance course (C) exceeds a threshold (D) and/or the measurement indicative of a disease status of the patient (14) exceeds a predefined tolerance limit (E).

**17.** System according to claim 15, wherein the remote monitoring device (28) is further configured to provide a bi-directional communication with the portable device (18) and to transmit an indication of a required treatment as a part of the signal (16) to the portable device (18) and/or a second remote device.

Fig. 1

Fig. 2

Fig. 3

Fig. 4

Fig. 5

Europäisches
Patentamt

European
Patent Office

Office européen
des brevets

## EUROPEAN SEARCH REPORT

Application Number

EP 19 19 6291

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/281630 A1 (SEKURA RONALD D [US]) 13 November 2008 (2008-11-13) * claims 9, 12; paragraphs [0003], [0014] - [0018], [0056] - [0062]; figure 1A * ----- | 1-17 | INV. G16H20/10 |
| A | Hexal Ag: "Pollenflug-App für Android und iPhone ¦ Allergie HEXAL", , 7 July 2019 (2019-07-07), XP055650284, Retrieved from the Internet: URL:https://web.archive.org/web/2019070711 4147id_/https://allergie.hexal.de/pollenfl ug/pollenflug-app/ [retrieved on 2019-12-06] * the whole document * ----- | 1-17 | |

TECHNICAL FIELDS
SEARCHED      (IPC)

G16H

The present search report has been drawn up for all claims

| Place of search | Date of completion of the search | Examiner |
|---|---|---|
| Munich | 18 December 2019 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 19 6291

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2019

| Patent document cited in search report | Publication date | Patent family member(s) | Publication date |
|---|---|---|---|
| US 2008281630 A1 | 13-11-2008 | US 2008281630 A1<br>US 2011196700 A1 | 13-11-2008<br>11-08-2011 |

EPO FORM P0459

For more details about this annex : see Official Journal of the European Patent Office, No. 12/82